# EUROPEAN PATENT APPLICATION

(11) **EP 2 157 356 A1**
(43) Date of publication of application: **24.02.2010**
(21) Application number: 08765120.4
(22) Date of filing: 04.06.2008
(51) Int. Cl.: F21S 2/00, F21V 14/00, H05B 37/04, A61B 1/06, F21W 131/205, F21Y 101/02, G03B 21/14

(54) **LIGHT SOURCE DEVICE FOR LIGHTING**

(30) Priority: 08.06.2007 JP 2007152954
(71) Applicant: Olympus Corporation, Tokyo 151-0072 (JP)
(72) Inventor: HANANO, Kazunari, Tokyo 151-0072 (JP)
(74) Representative: Schmidt, Steffen
(86) International application number: PCT/JP2008/060307
(87) International publication number: WO 2008/149905

(57) **Abstract**

A low-cost, high-reliability, high-luminance light source is provided without the need to provide a spare light source. An LED light source device (1) includes a plurality of LEDs (3); a light-guide optical unit (5) that guides illumination light emitted from the LEDs (3) to an illuminated region; a motor (7) that relatively moves the light-guide optical unit (5) and the LEDs (3); an LED lighting control circuit (33) that drives the LEDs (3) to blink in synchronization with the relative motion effected by the motor (7); a light-level sensor (9) that detects the light level of the illumination light guided to the illuminated region; and a fault determination unit (35) that stops the blinking driving effected by the LED lighting control circuit (33) and continuously lights all of the LEDs (3) when the light level of the illumination light detected by the light-level sensor (9) reaches or falls below a prescribed threshold.

## Description

### Technical Field

The present invention relates to an illumination light source device.

### Background Art

In the related art, there are known illumination light source devices that use LEDs (see Patent Document 1 and Patent Document 2). The illumination light source devices described in Patent Document 1 and Patent Document 2 obtain bright illumination light that appears to be continuous by instantaneously causing intense light emission at a prescribed period in individual LED light sources while moving a plurality of LED light sources at high speed.

Accordingly, although it is possible to obtain bright illumination light as well as to extend the lifetime of the LEDs, the illumination light source devices described in Patent Document 1 and Patent Document 2 suffer from the problem that, in the event of a malfunction in the light source etc., the light level fluctuates or the light level becomes zero.

In contrast, the technology described in Patent Document 3 is an illumination light source device in which the light level does not fluctuate in the event of a malfunction in the light source etc. In the illumination light source described in Patent Document 3, two light source lamps are provided, and by switching the orientation of a mirror from one light source to the other light source, replacement of a light source can be performed rapidly while the light sources are switched.
Patent Document 1:
   Japanese Unexamined Patent Application, Publication No. 2003-346503
Patent Document 2:
   Japanese Unexamined Patent Application, Publication No. 2003-024275
Patent Document 3:
   Japanese Unexamined Patent Application, Publication No. Hei-4-104583

### Disclosure of Invention

However, when applying the approach of providing a lamp for use in the event of an abnormality, disclosed in Patent Document 3 above, to the approach disclosed in Patent Document 1 and Patent Document 2, there is a problem in that a spare light source, such as a lamp or LED, for use in the event of an abnormality is provided separately from the plurality of LED light sources, causing the size and cost of the device to increase.

The present invention has been conceived in light of such a situation, and an object thereof is to provide a low-cost, high-reliability, high-luminance light source without the need to provide a spare light source.

In order to solve the problems described above, the present invention employs the following solutions.
A first aspect of the present invention is an illumination light source device including a plurality of light emitters; a light-guide member that guides illumination light emitted from the light emitters to an illuminated region; a rotatable portion that relatively moves the light-guide member and the light emitters; a blinking controller that drives the light emitters to blink in synchronization with the relative motion effected by the rotatable portion; a light-level sensor that detects the light level of the illumination light guided to the illuminated region; and a lighting controller to light in the case of malfunctions, the lighting controller stopping the blinking driving effected by the blinking controller and continuously lighting all of the light emitters, when the light level of the illumination light detected by the light-level sensor reaches or falls below a prescribed threshold.

With the first aspect, the light-guide member and the plurality of light emitters are relatively moved by the operation of the rotatable portion, and the light emitters are driven to blink in synchronization with the relative motion of the light-guide member. In other words, of the plurality of light emitters, illumination light emitted from the light emitter disposed at a position opposing the light-guide member is guided to the illuminated region; therefore, the relative motion of the light-guide member and the plurality of light emitters can be conducted at high speed, and it is possible to obtain bright illumination light that appears to be continuous by making the plurality of light emitters emit light in a sequence of single pulses one after another.

In this case, if the light level of the illumination light detected by the operation of the light-level sensor reaches or falls below a prescribed threshold, by operating the lighting controller to light in the case of malfunctions, the blinking driving by the blinking controller is stopped, and all of the light emitters are continuously lit. Therefore, even in the event of an abnormality such as a decrease or flickering (hereinafter called "flicker") of the light level, due to, for example, a phase shift between the relative motion effected by the rotatable portion and the blinking driving by the blinking controller, or due to the relative motion effected by the rotatable portion stopping, it is possible to introduce the illumination light emitted from one of the light emitters into the light-guide member and to continuously supply stable illumination light to the illuminated region. Accordingly, it is possible to ensure a stable, reliable light source without providing a spare light source for use in the event of an abnormality, which allows the costs to be lowered and the device to be made more compact.

A second aspect of the present invention is an illumination light source device including a plurality of light emitters; a light-guide member that guides illumination light emitted from the light emitters to an illuminated region; a rotatable portion that relatively moves the light-guide member and the light emitters; a blinking controller that drives the light emitters to blink in synchronization with the relative motion effected by the rotatable portion; a light-level sensor that detects the light level of the illumination light guided to the illuminated region; and an operation controller to operate in the case of malfunctions, wherein when the light level of the illumination light detected by the light-level sensor reaches or falls below a prescribed threshold, the operation controller stops the blinking driving effected by the blinking controller, continuously lights one of the light emitters, and stops the rotatable portion at an opposing position of the light-guide member and the light emitters where the light level detected by the light-level sensor is maximum.

With the second aspect, in the event of an abnormality, such as a malfunction in one of the light emitters, the light guide member is disposed at a position opposing the normal light emitter that is continuously lit; therefore, it is possible to continue emitting continuous illumination light without using the malfunctioning light emitter. Also, compared with the case where all of the light emitters are continuously lit, it is possible to achieve a saving in electrical power and to further reduce the load on the light emitters.

A third aspect of the present invention is an illumination light source device including a plurality of light emitters; a light-guide member that guides illumination light emitted from the light emitters to an illuminated region; a rotatable portion that relatively moves the light guide member and the light emitters; a blinking controller that drives the light emitters to blink in synchronization with the relative motion effected by the rotatable portion; a light-level sensor that detects the light level of the illumination light guided to the illuminated region; and an operation controller to operate in the case of malfunctions, wherein when the light level of the illumination light detected by the light-level sensor reaches or falls below a prescribed threshold, the operation controller stops the relative motion effected by the rotatable portion, sequentially lights the light emitters, and continuously lights the light emitter disposed at an opposing position of the light-guide member and the light emitters where the light level detected by the light-level sensor is maximum.

With the third aspect, in the event of an abnormality, for example, a malfunction in the rotatable portion, the light emitter at a position opposing the light-guide member is continuously lit; therefore, it is possible to continue to emit continuous illumination light without making the light-guide member and the light emitter move relatively. Also, compared with the case where all of the light emitters are continuously lit, it is possible to achieve a saving in electrical power and to further reduce the load on the light emitters.

The present invention affords an advantage in that it is possible to provide a low-cost, high-reliability, high-luminance light source without the need to provide a spare light source.

### Brief Description of Drawings

[FIG. 1A] Fig. 1A is a schematic structural diagram of an LED light source device according to a first embodiment of the present invention.
[FIG. 1B] Fig. 1B is a diagram showing the LED light source device in Fig. 1A from a direction along the axis.
[FIG. 2] Fig. 2 is a block diagram showing a controller in Fig. 1A and Fig. 1B.
[Fig. 3A] Fig. 3A is a diagram showing a state in which a motor in Fig. 1 is stopped.
[FIG. 3B] Fig. 3B is a diagram showing the LED light source device in Fig. 3A from a direction along the axis.
[FIG. 4A] Fig. 4A is a diagram showing a state in which all LEDs in Fig. 1 are DC lit.
[FIG. 4B] Fig. 4B is a diagram showing the LED light source device in Fig. 4A from a direction along the axis.
[FIG. 5A] Fig. 5A is a diagram showing the relationship between forward current (vertical axis) and time (horizontal axis) during pulsed lighting.
[FIG. 5B] Fig. 5B is a diagram showing the relationship between forward current (vertical axis) and time (horizontal axis) during DC lighting.
[FIG. 6A] Fig. 6A is a diagram showing the relationship between forward current (vertical axis) and time (horizontal axis) during pulsed lighting prior to light-level adjustment.
[FIG. 6B] Fig. 6B is a diagram showing the relationship between forward current (vertical axis) and time (horizontal axis) during pulsed lighting after light-level adjustment.
[FIG. 6C] Fig. 6C is a diagram showing the relationship between forward current (vertical axis) and time (horizontal axis) during pulsed lighting after light-level adjustment.
[FIG. 7] Fig. 7 is a block diagram showing a controller of an LED light source device according to a second embodiment of the present invention.
[FIG. 8A] Fig. 8A is a schematic structural diagram of the LED light source device in Fig. 7.
[FIG. 8B] Fig. 8B is a diagram showing the LED light source device in Fig. 8A from a direction along the axis.
[FIG. 9A] Fig. 9A is another schematic structural diagram of the LED light source device according to the second embodiment of the present invention.
[FIG. 9B] Fig. 9B is a diagram showing the LED light source device in Fig. 9A from a direction along the axis.
[FIG. 10] Fig. 10 is a flowchart for the LED light source device according to the second embodiment of the present invention.
[FIG. 11] Fig. 11 is a diagram showing a control device of an LED light source device according to a modification of the second embodiment of the present invention.
[FIG. 12] Fig. 12 is a diagram showing an LED light source device according to another modification of the second embodiment of the present invention.
[FIG. 13A] Fig. 13A is a schematic structural diagram of a Reference Example of an LED light source device.
[FIG. 13B] Fig. 13B is a diagram showing the LED light source device in Fig. 13A from a direction along the axis.
[FIG. 14A] Fig. 14A is another schematic structural diagram of the Reference Example of the LED light source device.
[FIG. 14B] Fig. 14B is a diagram showing the LED light source device in Fig. 14A from a direction along the axis.
[FIG. 15A] Fig. 15A is a schematic structural diagram of another Reference Example of an LED light source device.
[FIG. 15B] Fig. 15B is a diagram showing the LED light source device in Fig. 15A from a direction along the axis.
[FIG. 16A] Fig. 16A is another schematic structural diagram of the Reference Example in Fig. 15A and Fig. 15B.
[FIG. 16B] Fig. 16B is a diagram showing the LED light source device in Fig. 16A from a direction along the axis.
[FIG. 17] Fig. 17 is a diagram showing another Reference Example of an LED illumination light source device.
[FIG. 18] Fig. 18 is a diagram showing another Reference Example of an LED illumination light source device.
[FIG. 19] Fig. 19 is a flowchart for the Reference Example of the LED illumination light source device.

### Explanation of Reference Signs:

1: LED light source device (illumination light source device)
3: LED (light emitter)
5: light-guide optical unit (light-guide member)
7: motor (rotatable portion)
9: light-level sensor
33: LED lighting control circuit (blinking controller)
35: fault determination unit (lighting controller to light in the case of malfunctions)

### Best Mode for Carrying Out the Invention

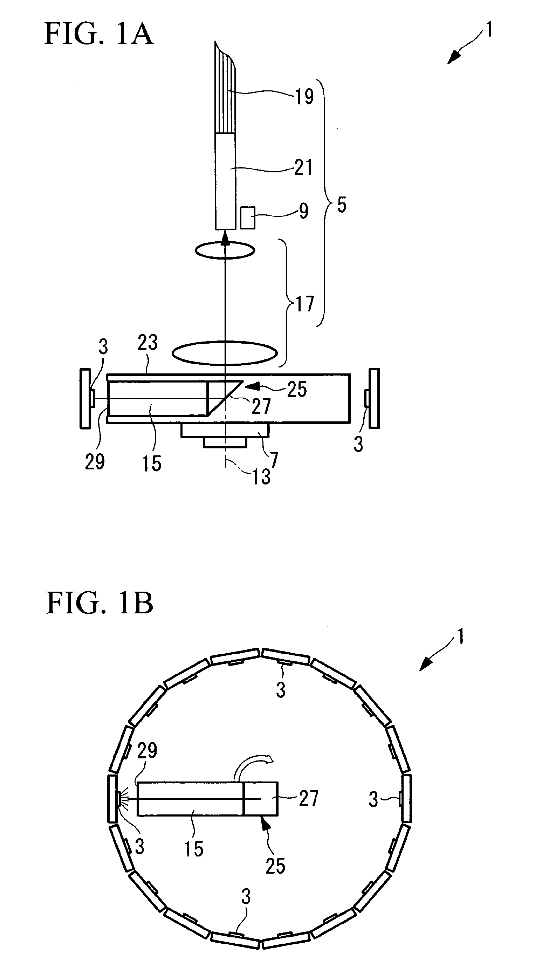

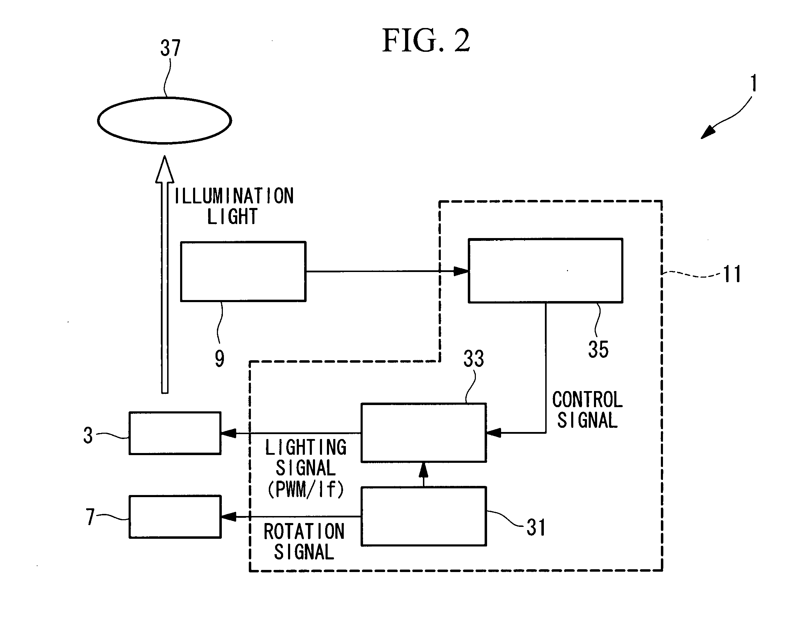

(First Embodiment) An LED light source device (illumination light source device) according to a first embodiment of the present invention will be described below with reference to the drawings.
As shown in Figs. 1A, 1B, and 2, the LED light source device 1 according to this embodiment includes a plurality of LEDs (light emitters) 3, a light-guide optical unit 5 that guides illumination light emitted from the LEDs 3 to an illuminated region, which is not shown in the drawings, a motor (rotatable portion) 7 that rotationally drives the light-guide optical unit 5, a light-level sensor 9 that detects the light level of the illumination light guided to the illuminated region, and a controller 11 that controls the LEDs 3 and the motor 7.

The LEDs 3 are disposed in the form of a ring centered on an axis 13. More specifically, 18 LEDs 3 are disposed adjacent to each other in the circumferential direction so as to directly oppose the axis 13.
The light-guide optical unit 5 includes a light-guide optical rod 15 which the illumination light from the LEDs 3 enters, relay lenses 17, and light-guide fibers 19 that guide the illumination light guided by the light-guide optical rod 15 and the relay lenses 17 towards an illumination unit, which is not shown in the drawings. Reference sign 21 indicates a single rod that relays the illumination light to be introduced into the light-guide fibers 19, and reference sign 23 indicates a hood that serves as an anti-wind-noise and anti-dust measure.

The light-guide optical rod 15 is formed in a cylindrical shape and is provided with a reflecting mirror 27 at an exit end 25. The light-guide optical rod 15 is disposed such that an entrance end 29 is disposed so as to be directed radially outward, and the reflecting mirror 27 at the exit end 25 is located on the axis 13. Accordingly, the illumination light emitted from the LED 3 at a position opposing the entrance end 29 is introduced into the light-guide optical rod 15, which guides the illumination light in the radial direction, and the illumination light is reflected in a direction along the axis 13 by the reflecting mirror 27 at the exit end 25.

The light-guide fibers 19 are for guiding the illumination light to the illumination unit. The end face of the single rod 21 provided at the tip of the light-guide fibers 19 is disposed on the axis 13, and the illumination light emitted from the exit end 25 of the light-guide optical rod 15 is introduced thereto via the relay lenses 17. A light-level sensor 9 is provided in the vicinity of the single rod 21 of the light-guide fibers 19 to detect the light level of the illumination light entering the light-guide fibers 19.

The motor 7, which has a rotating shaft that is coaxial with the axis 13, rotates the light-guide optical rod 15 about the axis 13 to move the entrance end 29 relative to the plurality of LEDs 3. The top surface and the bottom surface of the rotational range of the light-guide optical rod 15, in other words the inner side of the LEDs 3 arranged in a ring, are covered by the hood 23, thus preventing the occurrence of wind noise due to the rotation of the light-guide optical rod 15.

As shown in Fig. 2, the controller 11 includes a motor control circuit 31 that controls the rotational driving of the motor 7, an LED lighting control circuit (blinking controller) 33 that controls the lighting of the LEDs 3, and a fault determination unit (lighting controller to light in the case of malfunctions) 35 that determines the status of the device on the basis of the light level of the illumination light detected by the light-level sensor 9 and that controls the LED lighting control circuit 33. Reference sign 37 indicates a subject.

The operation of the thus-configured LED light source device 1 according to this embodiment will now be described.
With the LED light source device 1 according to this embodiment, a rotation signal is output from the motor control circuit 31 to rotationally drive the motor 7, thereby rotating the light-guide optical rod 15 about the axis 13.

The rotation signal from the motor control circuit 31 is also input to the LED lighting control circuit 33, and a lighting signal for lighting the LEDs 3 in synchronization with the rotational driving of the motor 7 is output from the LED lighting control circuit 33. Accordingly, of the plurality of LEDs 3 arranged in a ring, illumination light is emitted only from the LED 3 at a position opposing the entrance end 29 of the light-guide optical rod 15 and enters the light-guide optical rod 15.

The illumination light entering the light-guide optical rod 15 is deflected in a direction along the axis 13 by the reflecting mirror 27, is guided towards the light-guide fibers 19 via the relay lenses 17, is guided from the light-guide fibers 19 to the illumination unit, and is emitted towards the illuminated region.

With the LED light source device 1, because the rotation signal for high-speed rotational driving is output from the motor control circuit 31, and the lighting signal for lighting with pulsed light emission is output from the LED lighting control circuit 33, the LEDs 3 at positions opposing the entrance end 29 of the light-guide optical rod 15 that rotates at high speed emit light in a series of single pulses, and the illumination light sequentially enters the light-guide optical rod 15 to be guided towards the illumination unit; therefore, bright illumination light that appears to be continuous can be emitted from the illumination unit.

Accordingly, by making the individual LEDs 3 instantaneously emit intense light, a larger light level can be obtained compared with the case where the LEDs 3 are made to emit light continuously; therefore, it is possible to efficiently emit high-luminance illumination light.

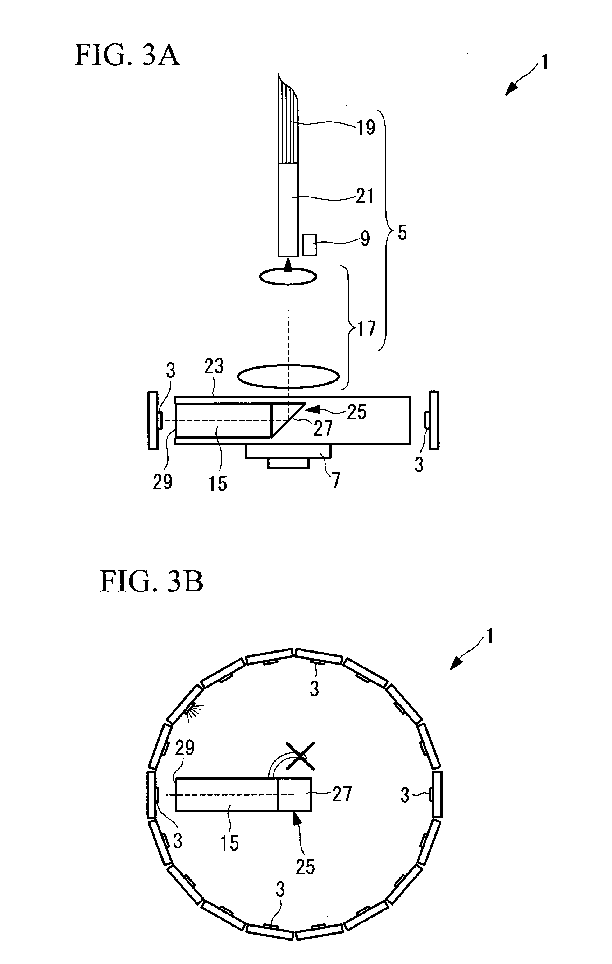

In the normal operating mode, the lighting of the LEDs 3 and the rotational driving of the motor 7 operate synchronously, as described above; however, as shown in Figs. 3A and 3B, if the rotational period becomes unstable (irregular rotation) or the rotation stops due to a malfunction in the motor 7, the lighting of the LEDs 3 and the light collection in the light-guide optical rod 15, in other words, the timing at which the LEDs are lit and the timing at which the motor 7 rotates, cannot be made to coincide, and the light level of the illumination light entering the light-guide fibers 19 thus drops.

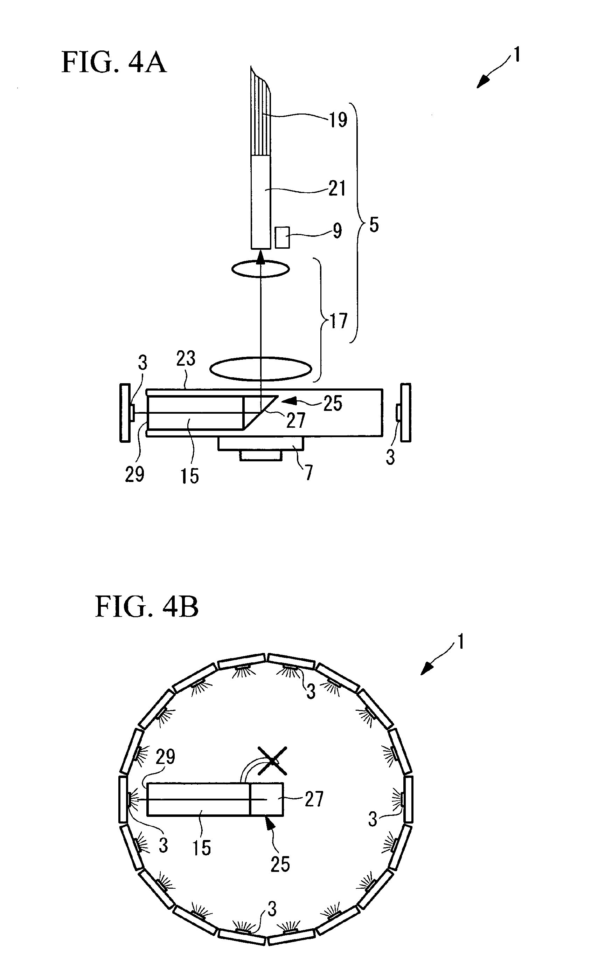

When the light level of the illumination light detected by the light-level sensor 9 reaches or falls below a prescribed threshold, by the operation of the fault determination unit 35, it is determined that the device is in an operation state in which a malfunction occurs, and the LED lighting control circuit 33 is controlled. More specifically, as shown in Figs. 4A and 4B, the LED lighting control circuit 33 is switched from pulsed light emission to continuous light emission (hereinafter referred to as "DC lighting"), and a lighting signal that DC lights all of the LEDs 3 is output.

Accordingly, whatever the position of the entrance end 29 of the light-guide optical rod 15, the illumination light emitted from one of the LEDs 3 can be introduced to the light-guide optical rod 15 and guided to the illumination unit. Therefore, stable illumination light can continue to be supplied to the illuminated region, and it is possible to prevent the light level from falling further or flickering.

As described above, with the LED light source device 1 according to this embodiment, it is possible to efficiently emit high-luminance illumination light and to ensure stability and reliability of the light source without providing a spare light source for use in the event of an abnormality, which makes it possible to lower costs and to reduce the size of the device.

In view of a design concept in which the occurrence of problems such as malfunctions, operating errors, design defects, etc., is anticipated and measures are provided such as those to minimize problems when an abnormality arises (hereinafter referred to simply as "failsafe"), for example, in the event of a malfunction in the light source, in some cases a spare light source is expected to have the same performance as the main light source, and in some case the spare light source need not fully satisfy the performance requirements of the main light source, but is classed only as a sub-light source and should have a performance that is sufficient as a primary backup for use in the event of an abnormality. Failsafe operation of the LED light source device 1 according to this embodiment is close to the latter idea, in consideration of cost-effectiveness.

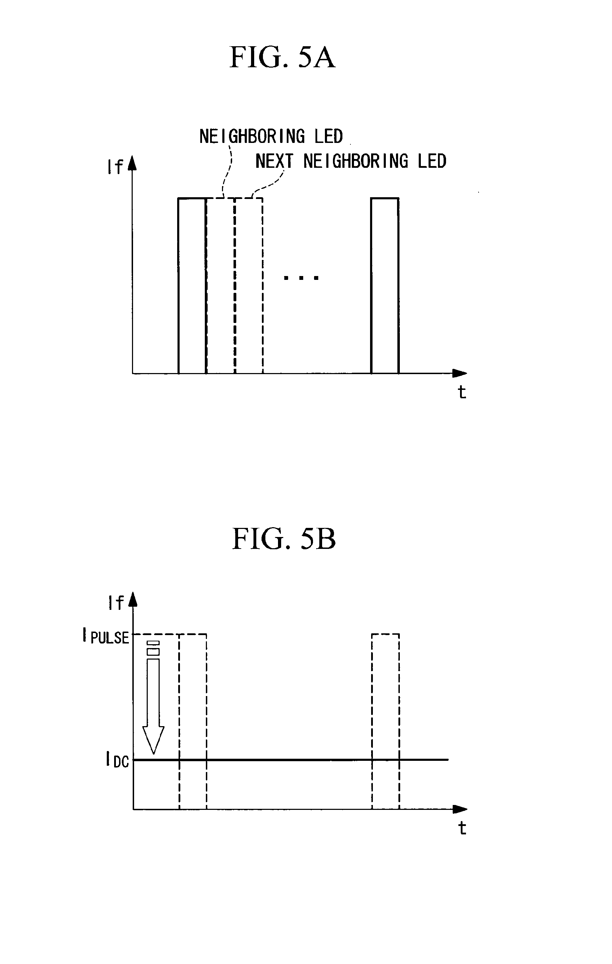

Therefore, with the LED light source device 1 according to this embodiment, although a current larger than the current during DC lighting is assumed to be applied to the individual LEDs 3 during normal operation, at the time of switching to DC lighting, it is preferable to reduce the light level of the illumination light from the viewpoint of lifetime and the temperature characteristics of the luminous efficiency. More specifically, the current values applied to the LEDs 3 in DC lighting are preferably reduced from those in the sequential pulsed lighting mode during normal operation, shown in Fig. 5A, to a rated current value or lower, as shown in Fig. 5B. In Figs. 5A and 5B, the vertical axis indicates forward current, and the horizontal axis indicates time.

Furthermore, in illumination systems for endoscopes, light-level adjustment processing according to the image-capture conditions is often performed to compensate for the dynamic range of the image-acquisition device. In other words, if the distance between the tip of the endoscope and the illuminated region changes, the brightness of the acquired image changes considerably; therefore, by increasing or decreasing the light level of the illumination light supplied to the illuminated region according to the luminance of the acquired image, the acquired image can be set to an appropriate luminance level when output on a monitor etc.

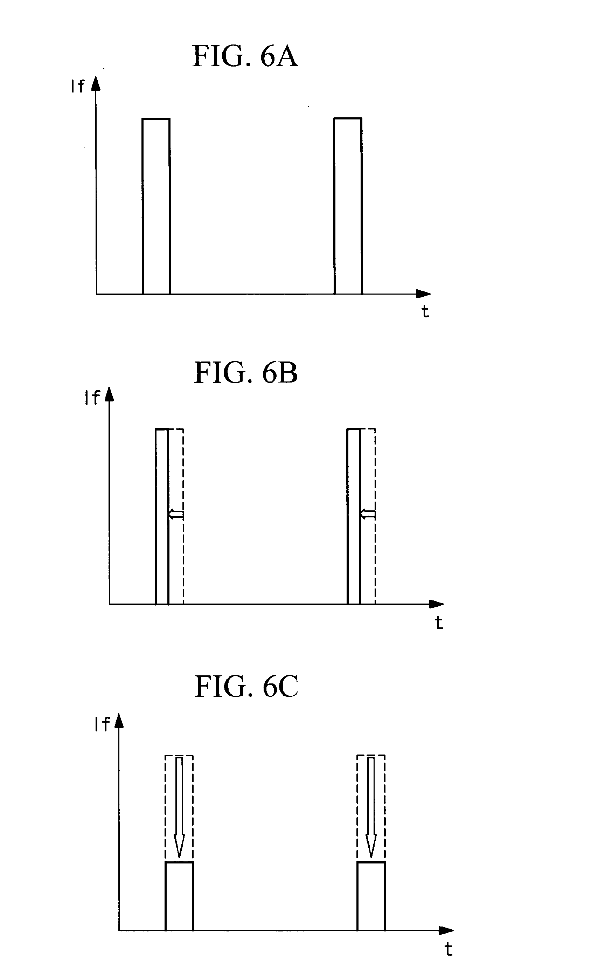

In this embodiment, because the LEDs 3 are used in the light source, in the normal mode where sequential pulsed lighting is performed, light level adjustment may be achieved by making the pulse width of the lighting signals applied to the individual LEDs 3 (see Fig. 6A) narrower, as shown in Fig. 6B, or by reducing the current, as shown in Fig. 6C. In either case, because the current is reduced as described above at the time of switching from sequential pulsed lighting to DC lighting, it is preferable to also match the changes in the light-level adjustment, as shown in Figs. 5A and 5B.

### (Second Embodiment)

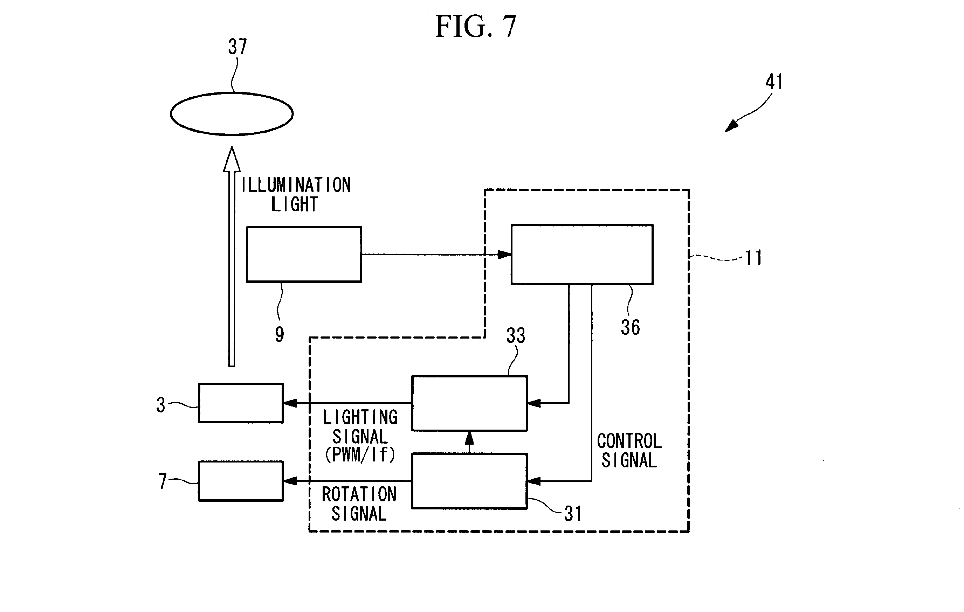

Next, an LED light source device 41 according to a second embodiment of the present invention will be described with reference to Figs. 7 to 10.
In the description of this embodiment, parts having the same configuration as those in the LED light source device 1 according to the first embodiment are assigned the same reference numerals, and a description thereof is omitted.

The LED light source device 41 according to this embodiment differs from the first embodiment in the way of controlling the motor 7 with the motor control circuit 31 and controlling the LEDs 3 with the LED lighting control circuit 33 during operation in the case of malfunctions. Specifically, in the first embodiment, it is assumed that the fault determination unit 35 controls only the LED lighting control circuit 33, whereas this embodiment differs in that, as shown in Fig. 7, a fault determination unit (operation controller to operate in the case of malfunctions) 36 controls the motor control circuit 31 in addition to the LED lighting control circuit 33.

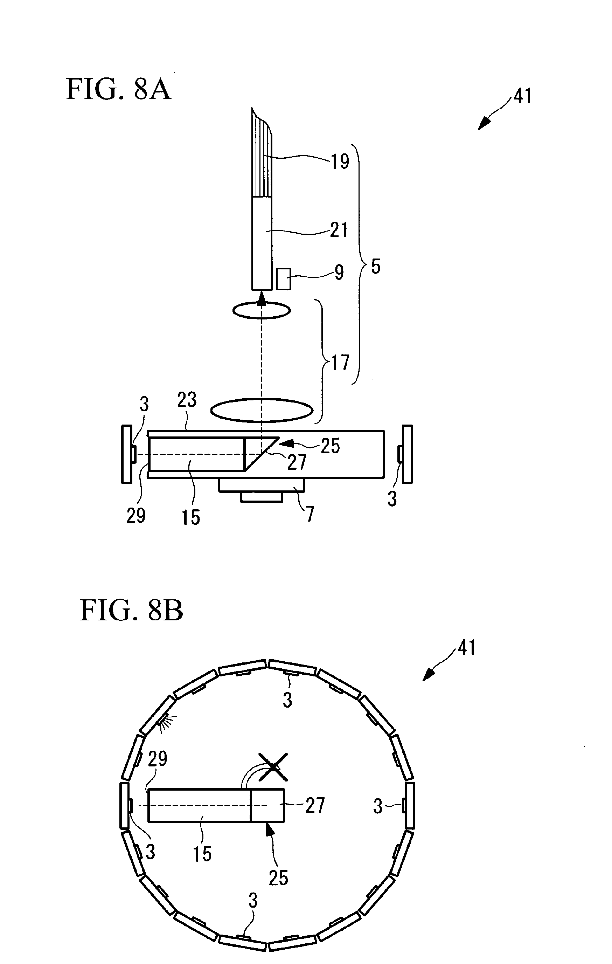

As shown, for example, in Figs. 8A and 8B, with the LED light source device 41 according to this embodiment, when the rotational period becomes unstable (irregular rotation) or rotation stops because of a malfunction in the motor 7, and an operation state in which a malfunction occurs is determined by the fault determination unit 36, a stop signal is output from the motor control circuit 31. Note that it is preferable to use a cogging (lock) type motor for the motor 7. Accordingly, it is possible to stop the rotation of the light-guide optical rod 15 at a position where the entrance end 29 opposes one of the LEDs 3.

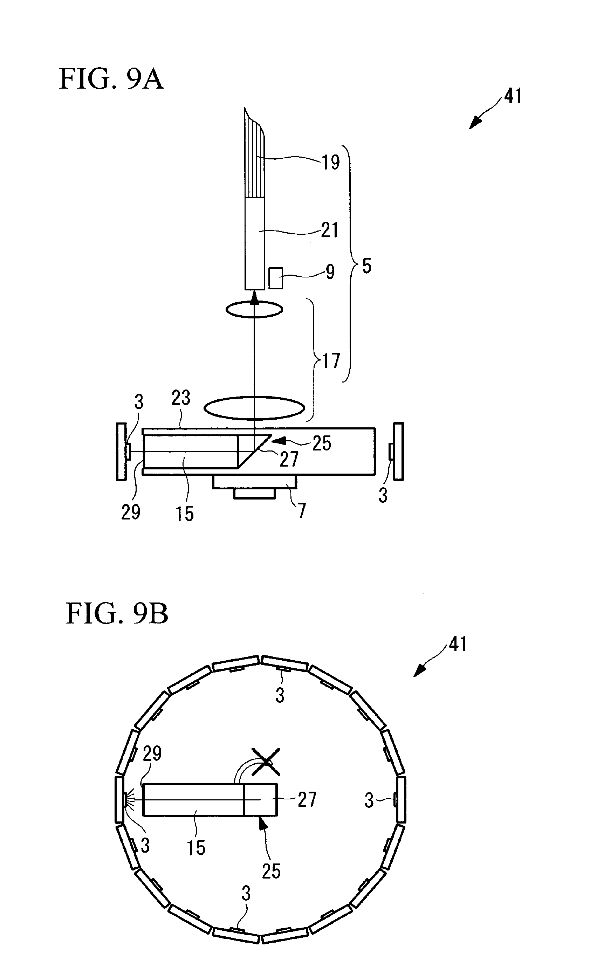

A lighting signal that sequentially lights each of the LEDs 3 in turn is output from the LED lighting control circuit 33. Thus, when the LED 3 at a position opposing the entrance end 29 of the stopped light-guide optical rod 15 is lit, the illumination light level detected by the light-level sensor 9 is maximum; therefore, as shown in Figs. 9A and 9B, only the LED 3 which gives the maximum output from the light-level sensor 9 is DC lit by the LED lighting control circuit 33, and the other LEDs 3 are turned off.

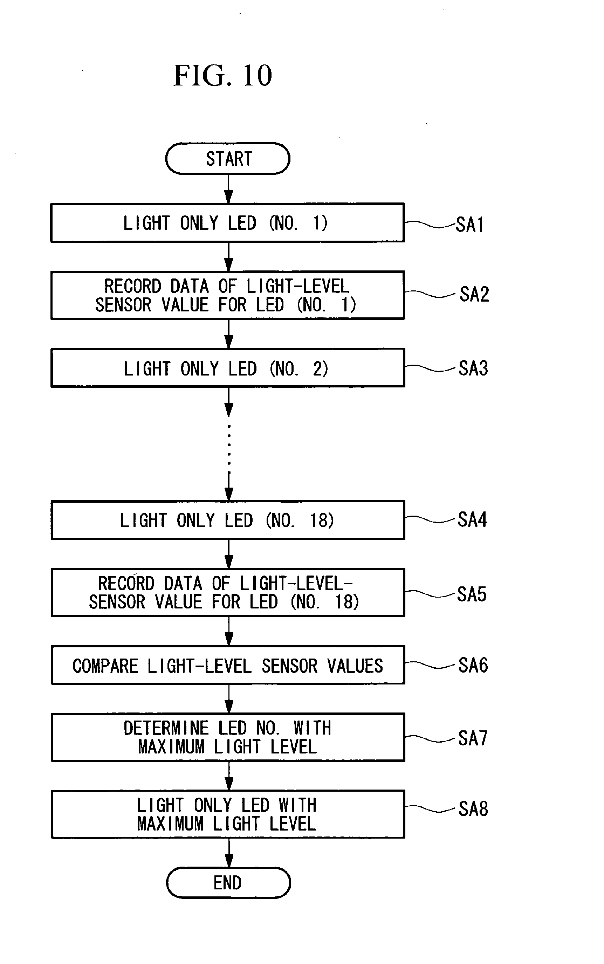

More specifically, as shown in Fig. 10, first only one of the LEDs (No. 1) is lit (step SA1), and the light-level sensor value for the LED (No. 1) is stored in the fault determination unit 35 (step SA2). Then, for example, only the adjoining left-neighbor LED (No. 2) is lit (step SA3), and the light-level sensor value for the LED (No. 2) is stored in the fault determination unit 35, in the same way as in step SA3.

After one complete circuit, only the final LED (No. 18) is lit (step SA4), and the light-level sensor value thereof is stored in the fault determination unit 35 (step SA5), whereupon the light-level sensor values of all LEDs (No. 1) to (No. 18) in the fault determination unit 35 are compared (step SA6), and the LED No. with the maximum light level is determined (step SA7). Accordingly, the LED lighting control circuit 33 outputs a lighting signal that lights only the determined LED 3, and only the LED 3 with the maximum light level is lit (step SA8). Note that the processing for determining the LED 3 with the maximum output in this way may be installed in advance in the fault determination unit 35 in the form of a single sequence as a processing mode for malfunctions.

By doing so, it is possible to effectively deduce the direction in which the entrance end 29 of the light-guide optical rod 15 is facing. Then, because the LED 3 at a position opposing the light-guide optical rod 15 is DC lit, even though it is not possible to move the light-guide optical rod 15 and the LEDs 3 relative to each other, it is possible to continue supplying continuous illumination light to the illuminated region.
Also, because one of the LEDs 3 is DC lit, it is possible to use less electrical power and to reduce the load on the light emitter compared with a case in which all of the LEDs 3 are DC lit.

Note that, in this embodiment, during operation in the case of malfunctions, it is assumed that only the LED 3 that is desired to be discriminated is DC lit; instead of this, however, a group of multiple LEDs 3, also including neighboring LEDs 3, for example, may be DC lit.
In this embodiment, the light-level sensor 9 for detecting the light level of the illumination light has been described as an example; instead of this, however, the light level of the illumination light may be determined from, for example, the luminance value of the acquired image signal. In this case, by sequentially lighting the LEDs 3 in turn while maintaining fixed image acquisition conditions, it can be determined that the LED 3 having illumination light with the maximum luminance value is positioned closest to the entrance end 29 of the light-guide optical rod 15.

The LED light source device 41 according to this embodiment can be modified as follows.
For example, if one of the LEDs 3 cannot be lit due to a malfunction, or if the light level of the illumination light fluctuates, causing the light-level sensor value to reach or fall below a prescribed threshold, the fault determination unit 36 may control the LED control circuit 33 and the motor control circuit 31 as follows. Specifically, the LED lighting control circuit 33 may switch from pulsed light emission to DC lighting, whereupon one of the LEDs 3 is DC lit, and the motor control circuit 31 may control the rotational driving of the motor 7 so as to stop the motor 7 at the opposing position of the light-guide optical rod 15 and the LED 3 where the light level of the illumination light detected by the light-level sensor 9 is maximum.
By doing so, the entrance end 29 of the light-guide optical rod 15 is disposed at a position opposing the LED 3 that is DC lit; therefore, the malfunctioning LED 3 is not used, and continuous illumination light can continue to be emitted.

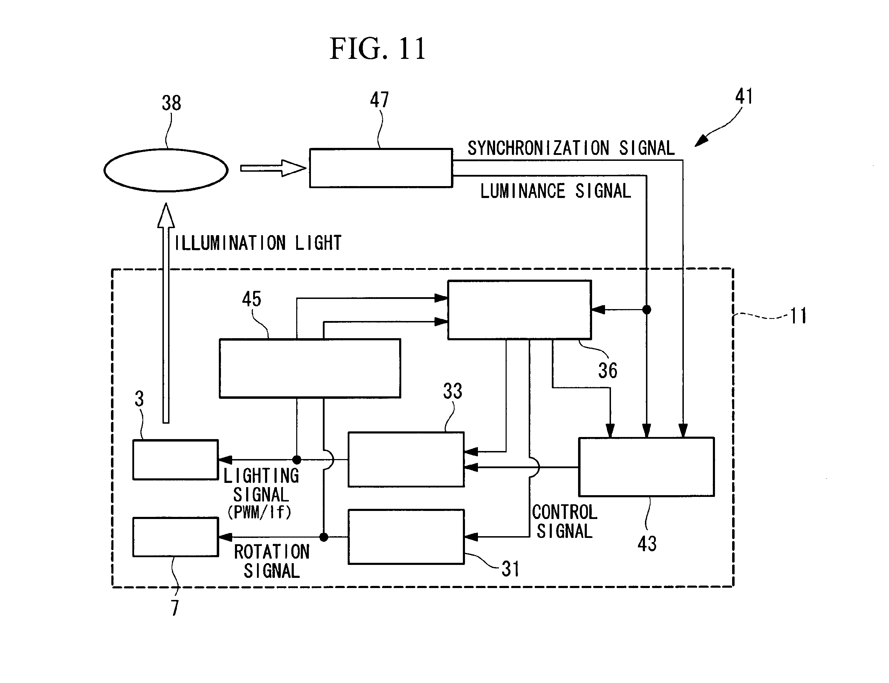

As shown in Fig. 11, instead of the sensor 9, the controller 11 may be provided with a light-level-adjustment control circuit 43 and lighting-state detecting means 45 and may perform control and adjustment of the light level of the illumination light emitted from the LEDs 3 based on the level of a luminance signal obtained by acquiring the illuminated subject 37 with an image-acquisition device 47.
In this case, in order to synchronize image acquisition with the image-acquisition device 47 and light emission of illumination light from the LEDs 3, a luminance signal and a synchronization signal are output from the image-acquisition device 47 to the light-level-adjustment control circuit 43. The luminance signal should also be input to the fault determination unit 35, and in addition, current, phase, and period, or position etc., should be detected in the lighting-state detecting means 45 on the basis of the lighting signal from the LED lighting control circuit 33 and the rotation signal from the motor control circuit 31, and these various kinds of signals should be input to the fault determination unit 36, where it is determined whether the operating state of the device is normal or faulty. Then, if it is determined that the operating state of the device is faulty, the motor control circuit 31 and the LED lighting control circuit 33 should be controlled by the fault determination unit 36 and the light-level-adjustment control circuit 43, and processing should be carried out whereby all LEDs 3 are DC lit or one of the LEDs 3 is DC lit, as described above.
In this modification, the luminance signal has been described as an example of the input signal for setting the light-adjustment level; instead of this, however, the light-adjustment level may be set by monitoring the output from the light-level sensor 9.

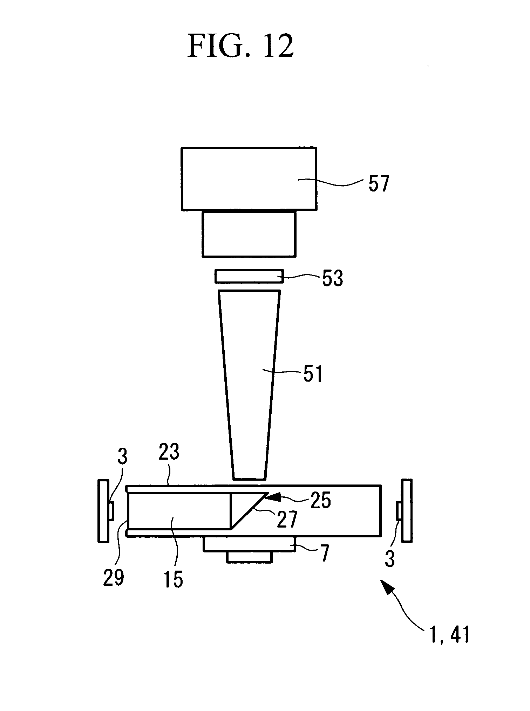

In a modification in which the LED light source device 1 or 41 according to the above-described embodiments is applied to a projector, as shown in Fig. 12, the illumination light emitted from the LED light source device 1 or 41 is guided to a transmissive LED panel 53 by an illumination optical rod 51 and is modulated on the basis of a video signal, after which it is magnified and projected onto a screen by a projection lens 57. For example, in products referred to as rear projectors which are integrated with screens, there are certain monitoring and control applications in which lamp breakdowns are not permitted. Thus, the LED light source devices 1 and 41 according to the present invention, having the above-described fail-safe function, are effective in applications requiring stable and reliable light sources.

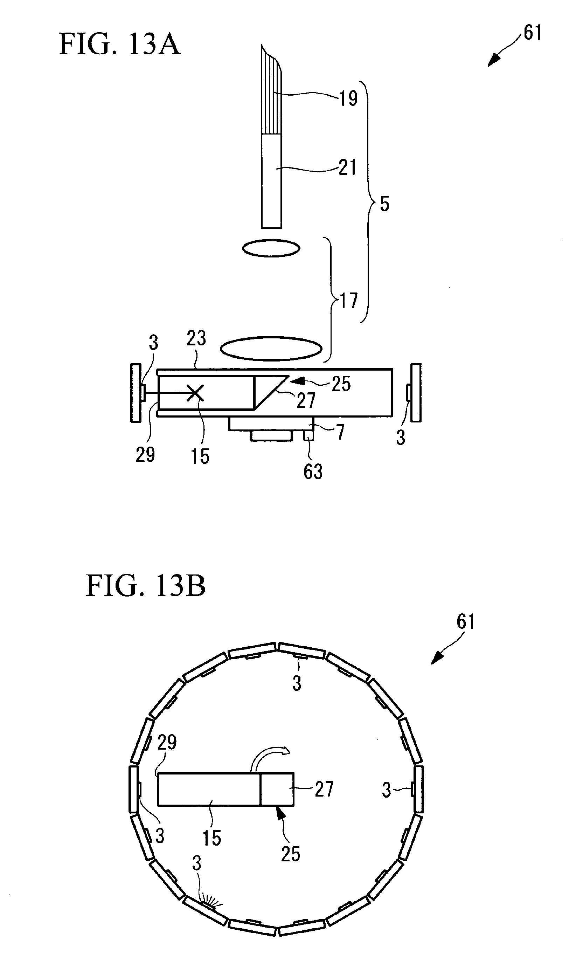

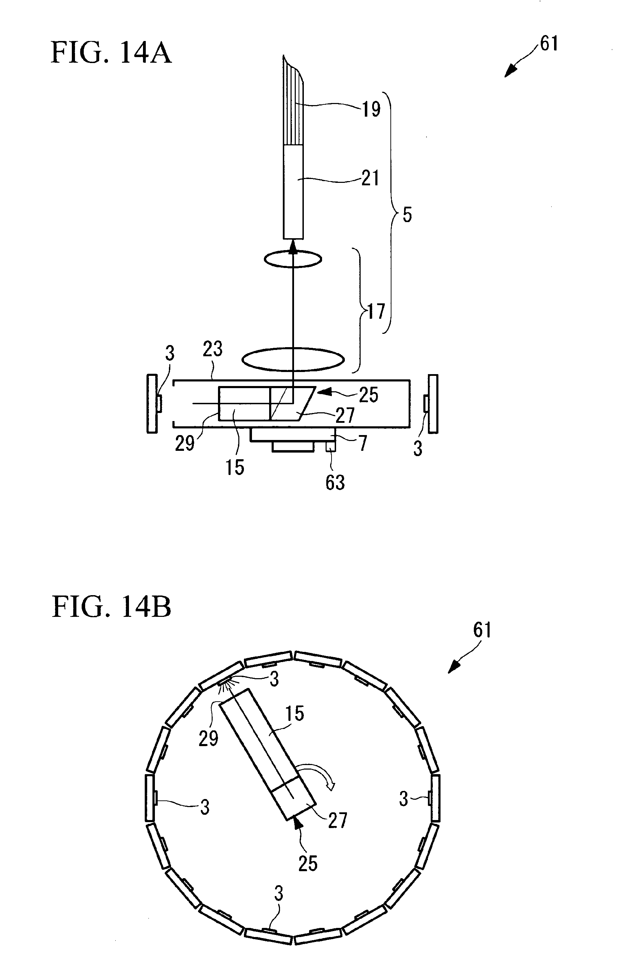

Apart from the LED light source devices 1 and 41 according to the above-described embodiments, the Reference Examples described below are available as fail-safe measures for operation in the case of malfunctions of illumination light source devices.
For example, as shown in Figs. 13A and 13B, in an LED light source device 61, the motor 7 is provided with an encoder 63 for detecting the phase, and when the light-emission timing of the LEDs 3 and the rotation timing of the motor 7 are shifted, as shown in Figs. 14A and 14B, in the case of either a phase shift or a period shift, the phase or period should be checked, and processing such as resetting, adjustment, etc. should be performed so as to realign the timings.

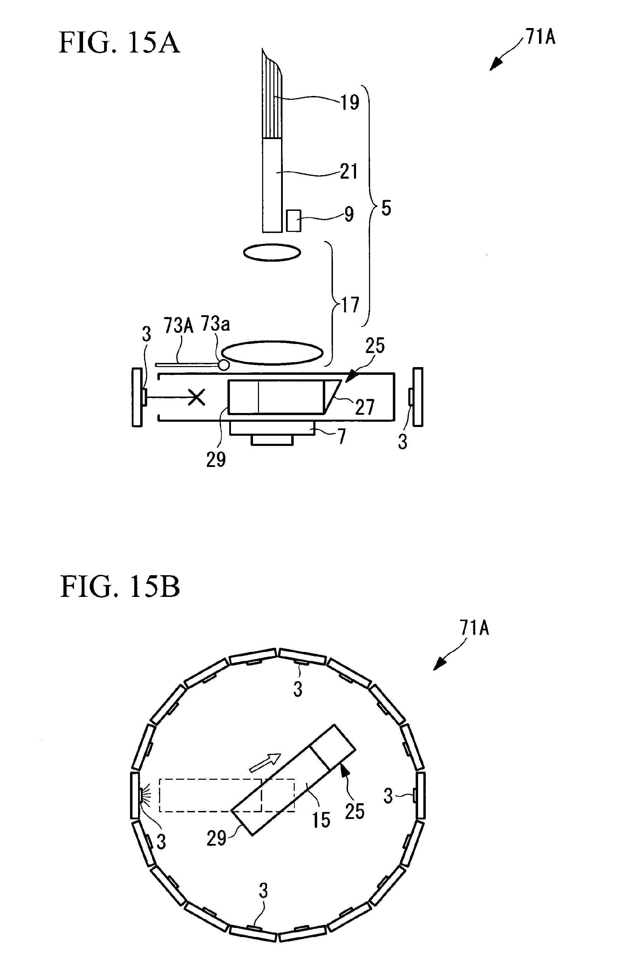

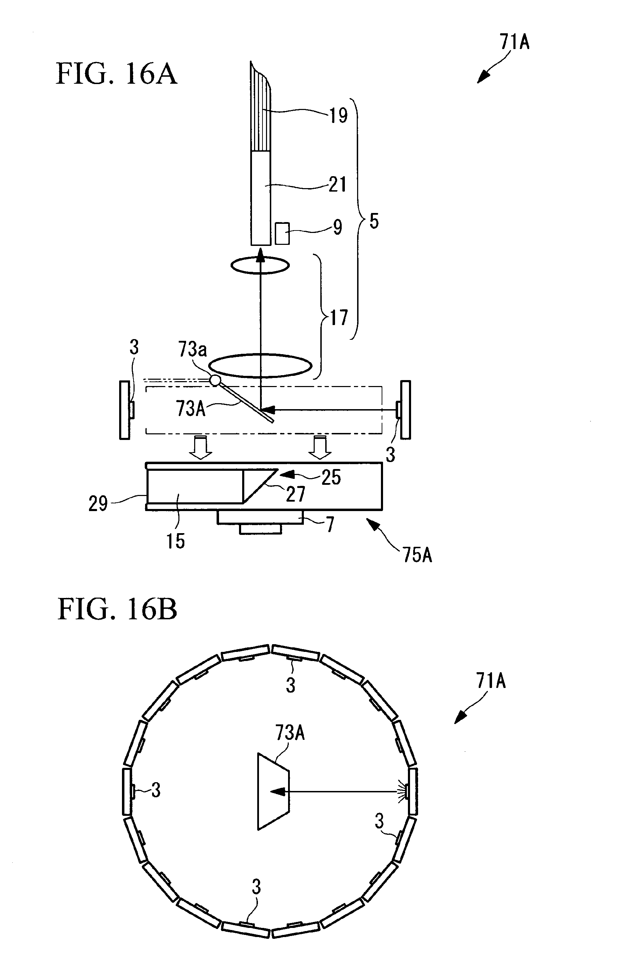

As shown in Figs. 15A and 15B, if the light-guide optical rod 15 deviates or breaks due to some problem with the motor 7, it becomes physically impossible to take in and guide a prescribed amount of light in the light-guide optical rod 15. Therefore, as shown in Figs. 16A and 16B, for example, an LED light source device 71A is provided with a mirror for use in the case of malfunctions (malfunction mirror) 73A that can be moved to the position at which the reflecting mirror 27 is installed by means of a hinge mechanism 73a; in addition, a rotating portion 75A, including the motor 7 and the light-guide optical rod 15, should be arranged in advance as an attachable/detachable structure, and upon removing the rotating portion 75A, the malfunction mirror 73A should be installed in the light path of the illumination light. Accordingly, by DC lighting the LED 3 at a position directly opposite the malfunction mirror 73A, it is possible to employ it as a temporary backup light source in the event of an abnormality, although the brightness of the illumination light is reduced.

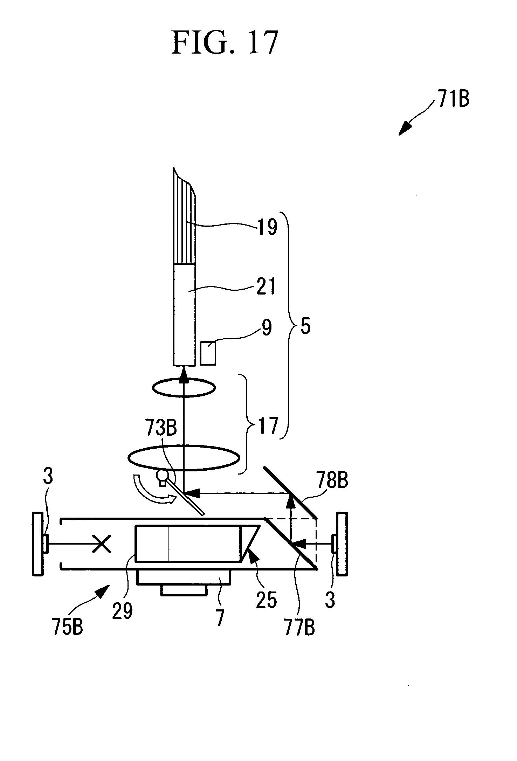

Instead of the method in which the above-described rotating portion 75A is removed, as shown in Fig. 17, for example, an LED light source device 71B is preferably provided with second malfunction mirrors 77B and 78B in a portion of the hood 23 that covers the light-guide optical rod 15, and the illumination light emitted from the LED 3 may be reflected towards the malfunction mirror 73B, which is attached to the central portion, via these second malfunction mirrors 77B and 78B to be guided towards the light-guide fibers 19. In this case, in order to ensure space for attaching the malfunction mirror 73B at the central portion, it is preferable to design a large gap between the rotating portion 75B and the relay lenses 17. In addition, because there is also a possibility of a malfunction in the LED 3 directly facing the malfunction mirror 73B, the malfunction mirror 73B is preferably of a two-axis movable type which is capable of rotating with the direction perpendicular to the axis 13 serving as an axis of rotation and which is capable of rotating with the axis 13 serving as an axis of rotation, so that the malfunction mirror 73B can be placed directly facing any LED 3.

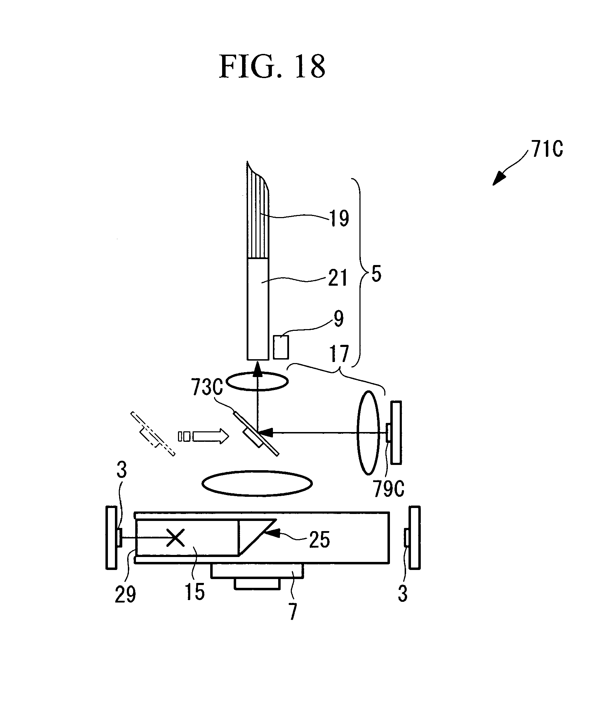

As shown in Fig. 18, an LED light source device 71C may be provided with a spare LED 79C for use in the event of an abnormality, separately from the plurality of LEDs 3 which are the main light sources, and in the event of an abnormality, a malfunction mirror 73C may be disposed in the light path, and the spare LED 79C may be lit.

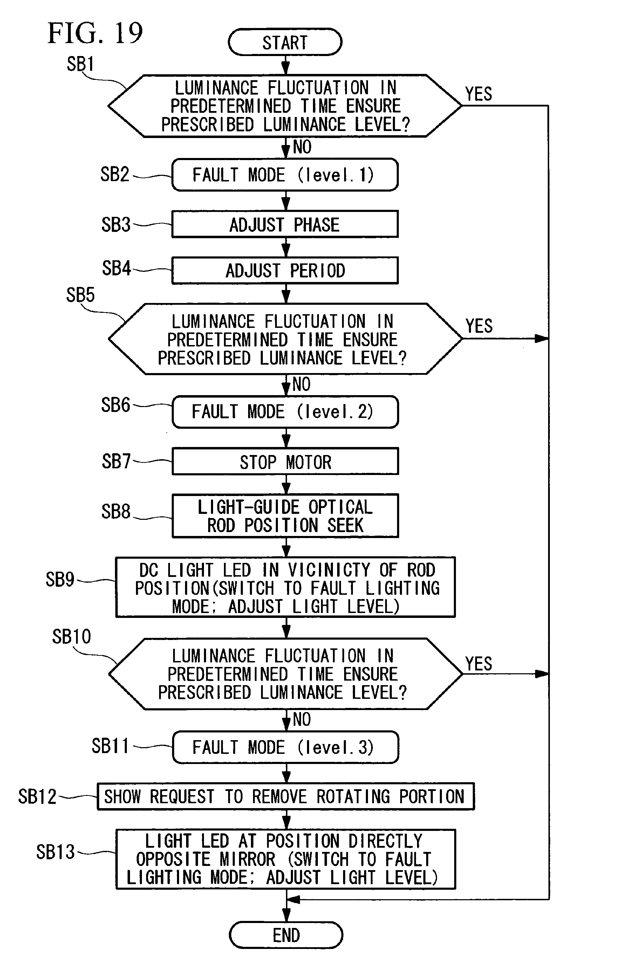

Next, processing at the time of operation in the case of malfunctions when the LED light source device 41 according to the above-described embodiment of the present invention is applied to the Reference Examples will be described using Fig. 19.
Normally, when the LED light source device 1 is lit in a sequential pulsed fashion to realize the light modulation function, in response to luminance fluctuation demands for increasing or reducing the light level, an appropriate light level according to the luminance fluctuation in a predetermined time is supplied so that a prescribed luminance level can be ensured ("YES" at step SB1). However, when a fault occurs, the luminance fluctuation demand cannot be satisfied and the appropriate light level cannot be supplied. In many cases, not being able to increase the light level, that is to say, not being able to ensure sufficient brightness, is more critical than reducing the light level.

For example, if an input signal is used to determine whether the luminance signal is normal or faulty, when a constant luminance level cannot be ensured for a predetermined time, in other words, when the luminance signal does not reach or exceed a prescribed value ("NO" at step SB1) regardless of the fact that control is performed by the LED lighting control circuit 33 to increase the luminance based on the control signal from the light-level-adjustment control circuit 43 (see Fig. 11), it is determined in the fault determination unit 36 that the operation is faulty, and fault mode (level.1) processing commences (step SB2). In this case, the rotational phase of the motor 7 is detected by the phase detecting encoder 63 (see Fig. 13A and Fig. 14A), and the period is calculated from the value thereof. Then, by comparison with the phase of light emission by the LEDs 3, the phase and period are adjusted (steps SB3 and SB4). For example, by matching the rotation timing of the motor 7 and the light-emission timing of the LEDs 3, the function of the LED light source device 1 is recovered, and it is possible to perform appropriate illumination ("YES" at step SB5). Note that, for adjustment of the phase and period, the light emission timing of the LEDs 3 may be made to match the rotation timing of the motor 7.

If the rotating portion 75 is stopped, worsening the luminance fluctuation, and the luminance signal still remains low even though the processing described above is performed, in other words, if a prescribed luminance level cannot be ensured ("NO" at step SB5), fault mode (level.2) processing commences in the fault determination unit 36 (step SB6), and the motor 7 is immediately stopped (step SB7). In this case, if the motor 7 is rotating irregularly, the rotation is stopped, and if the motor 7 is stopped due to a malfunction in the motor 7 itself even though the rotation signal for the motor 7 is ON, the rotation signal from the motor control circuit 31 should be turned OFF.

Next, the plurality of LEDs 3 are sequentially lit one by one (step SB8), and without operating the light-level-adjustment control circuit 43, only a group of LEDs or the LED 3 having the luminance signal with the highest output is lit (step SB9), and the other LEDs 3 are not lit. In this case, compared with the case in which the device functions normally with sequential pulsed lighting, the light level that can be supplied to the illuminated region is lower; therefore, the control signal from the light-level-adjustment control circuit 43 also changes simultaneously according to the electrical current value of the lighting signal. Accordingly, it is possible to continuously supply stable illumination light to the illuminated region ("YES" at step SB10).

If the luminance level still does not reach the prescribed level even though these processes are performed ("NO" at step SB10), a malfunction in the rotating portion 75 must be considered, and thus, fault mode (level.3) processing commences in the fault determination unit 36 (step SB11); for example, an indicator or the like should be immediately lit to request that the rotating portion 75 be removed (step SB12). Accordingly, the rotating portion 75 should be removed from the LED light source device 1, the malfunction mirror 73 should be installed, only the LED 3 directly facing the malfunction mirror 73 should be DC lit, and the other LEDs 3 should be turned off (step SB12). Thus, in the event of various types of malfunctions, by taking measures appropriate to the malfunction level, it is possible to continuously supply illumination light to the illuminated region.
Note that in this Reference Example, when the user determines that the operation is faulty for "NO" at step SB1, "NO" at step SB5, and "NO" at step SB10, the fault mode processing may be commenced by pushing a button or the like. In addition, instead of the process for DC lighting one of the LEDs 3, a process for DC lighting all of the LEDs 3 may be performed.

Although the embodiments of the present invention have been described above with reference to the drawings, the specific configurations are not limited to these embodiments, and the present invention also encompasses various design modifications so long as they do not depart from the spirit of the invention.

## Claims

1. An illumination light source device comprising:
a plurality of light emitters;
a light-guide member that guides illumination light emitted from the light emitters to an illuminated region;
a rotatable portion that relatively moves the light-guide member and the light emitters;
a blinking controller that drives the light emitters to blink in synchronization with the relative motion effected by the rotatable portion;
a light-level sensor that detects the light level of the illumination light guided to the illuminated region; and
a lighting controller to light in the case of malfunctions, the lighting controller stopping the blinking driving effected by the blinking controller and continuously lighting all of the light emitters, when the light level of the illumination light detected by the light-level sensor reaches or falls below a prescribed threshold.

2. An illumination light source device comprising:
a plurality of light emitters;
a light-guide member that guides illumination light emitted from the light emitters to an illuminated region;
a rotatable portion that relatively moves the light-guide member and the light emitters;
a blinking controller that drives the light emitters to blink in synchronization with the relative motion effected by the rotatable portion;
a light-level sensor that detects the light level of the illumination light guided to the illuminated region; and
an operation controller to operate in the case of malfunctions, wherein when the light level of the illumination light detected by the light-level sensor reaches or falls below a prescribed threshold, the operation controller stops the blinking driving effected by the blinking controller, continuously lights one of the light emitters, and stops the rotatable portion at an opposing position of the light-guide member and the light emitters where the light level detected by the light-level sensor is maximum.

3. An illumination light source device comprising:
a plurality of light emitters;
a light-guide member that guides illumination light emitted from the light emitters to an illuminated region;
a rotatable portion that relatively moves the light guide member and the light emitters;
a blinking controller that drives the light emitters to blink in synchronization with the relative motion effected by the rotatable portion;
a light-level sensor that detects the light level of the illumination light guided to the illuminated region; and
an operation controller to operate in the case of malfunctions, wherein when the light level of the illumination light detected by the light-level sensor reaches or falls below a prescribed threshold, the operation controller stops the relative motion effected by the rotatable portion, sequentially lights the light emitters, and continuously lights the light emitter disposed at an opposing position of the light-guide member and the light emitters where the light level detected by the light-level sensor is maximum.
